# EUROPÄISCHE PATENTSCHRIFT

(11) **EP 3 451 945 B1**
(45) Veröffentlichungstag und Bekanntmachung des Hinweises auf die Patenterteilung: **28.10.2020**
(21) Anmeldenummer: 17724769.9
(22) Anmeldetag: 02.05.2017
(51) Int. Cl.: A61B 17/15

(54) **UNTERKIEFERRESEKTIONSSCHABLONE**
MANDIBULAR RESECTION TEMPLATE
GABARIT DE RÉSECTION DE MÂCHOIRE INFÉRIEURE

(30) Priorität: 06.05.2016 DE 102016108433
(43) Veröffentlichungstag der Anmeldung: 13.03.2019
(73) Patentinhaber: Karl Leibinger Medizintechnik GmbH & Co. KG, 78570 Mühlheim (DE)
(72) Erfinder: HERZOG, Rebecca, 78570 Mühlheim (DE); GABELE, Lorenz, 78570 Mühlheim (DE)
(74) Vertreter: Winter, Brandl, Fürniss, Hübner, Röss, Kaiser, Polte - Partnerschaft mbB
(86) Internationale Anmeldenummer: PCT/EP2017/060433
(87) Internationale Veröffentlichungsnummer: WO 2017/191140

(56) Entgegenhaltungen:
- EP-A1- 3 000 439
- EP-A2- 2 792 329
- WO-A1-2004/039266
- CN-U- 204 863 356
- GB-A- 2 324 470
- US-A1- 2010 106 197
- US-A1- 2013 304 075

## Beschreibung

Die Erfindung betrifft eine Unterkieferresektionsschablone mit einem Zentralbauteil, das zum Anbringen an einem Segment, etwa einem Symphysensegment eines Kieferknochens, wie eines Unterkiefers oder eines Oberkiefers vorbereitet ist, wobei an dem Zentralbauteil mindestens zwei Trennwerkzeugführabschnitte vorhanden sind.

Aus dem Stand der Technik sind bereits Sägeschablonen, wie Unterkieferresektionsschablonen bekannt. So offenbart bspw. die WO 2004/039266 A1 eine Sägeschablone, die an einer Fibula oder einer Mandibula einsetzbar ist. Ähnliche Vorrichtungen sind auch aus der US 2012 /0029646 A1, der US 2013/0338779 A1 und der US 2013/0304075 A1 bekannt.

Eine weitere Unterkieferresektionsschablone ist in GB 2 324 470 A offenbart.

Es kommt vor, dass bspw. menschliche Unterkieferknochen, wie eine Mandibula durch einen Unfall oder karzinogene Veränderungen derart beschädigt ist, dass Teile dieses Knochens entnommen werden müssen. Der Knochen ist danach wieder zu vervollständigen. In jüngster Zeit wurde übergegangen Knochenabschnitte, die bspw. einer Fibula, also einem Wadenbein entnommen wurden, wieder an die defektbefreite Stelle / Leerstelle des Unterkiefers einzusetzen. Dazu besteht Bedarf, präzise Schnitte in vorbestimmter Weise sowohl am Unterkieferknochen und dann dazu korrespondierend am Wadenbein durchzuführen.

Hierfür werden üblicherweise Unterkieferresektionsschablonen eingesetzt, die also für eine präzise Schnittführung am Unterkiefer zuständig sind, genauso wie Fibula-Knochenmaterialentnahme- und -verbringschablonen, die für präzise Schnitte am Wadenbein zuständig sind und gleichzeitig für ein präzises Verbringen der ausgeschnittenen Knochen zum Implantieren im Kieferbereich eingesetzt sind.

Die bisher bekannten Lösungen sind nicht ausreichend präzise, obwohl sie schon sehr umständlich zu handhaben sind. Auch sind sie relativ kostenintensiv. Hier gilt es eine Verbesserung zu erreichen.

Es ist also die Aufgabe der vorliegenden Erfindung, die Nachteile aus dem Stand der Technik abzustellen oder zumindest zu mildern und eine kostengünstige Unterkieferresektionsschablone zur Verfügung zu stellen, die im Zusammenspiel mit einer Fibula-Knochenmaterialentnahme- und -verbringschablone so eingesetzt werden kann, dass ein ästhetisch und mechanisch ansprechendes Ergebnis im Unterkieferbereich erzielt werden kann.

Vereinzelt wird für Unterkiefer eine spezielle patientenspezifische Schablone angefertigt. Dies ist aber sehr kostenintensiv und zeitaufwändig, weswegen es der Ansatz der vorliegenden Erfindung ist, eine universelle Unterkieferresektionsschablone zur Verfügung zu stellen, die letztlich angepasst ist auf einen repräsentativen Patienten aus der Masse aller potenziellen Patienten.

Es sollen insbesondere universelle Schablonen zur Resektion des Unterkiefers und der Fibula bei anstehender Unterkieferrekonstruktion des mikrovaskulären Fibula-Transplantats sowie ein auf diese Technik abgestimmtes System an Miniplatten zur Transplantatfixierung zur Verfügung gestellt werden.

Diese Aufgabe wird durch eine Unterkieferresektionsschablone gemäß Anspruch 1 gelöst. Es ist vorgesehen, dass zwischen den beiden oder mehreren Trennwerkzeugführabschnitten, etwa nach Art von Sägeblattführabschnitten, eine Positionierhilfe vorhanden ist, um eine raumkorrekte Ausrichtung der Unterkieferresektionsschablone zum Kieferknochen zu bewerkstelligen. Auf diese Weise kann nämlich die Unterkieferresektionsschablone exakter und problemloser als bisher am Schädelknochen, insbesondere einem Kieferknochen, bevorzugt dem Unterkieferknochen des spezifischen Patienten befestigt werden, sodass eine hochpräzise Entnahme der zu entfernenden Knochenabschnitte durchgeführt werden kann.

Im Rahmen des erfindungsgemäßen Projektes wurden universelle Schablonen zur Resektion des Unterkiefers und der Fibula entwickelt. Die Intention war es nicht nur, eine deutliche Vereinfachung und Standardisierung des klinischen Eingriffs der Unterkieferrekonstruktion mittels mikrovaskulärem Fibula-Transplantats zu erreichen, sondern auch eine Kosten- und Zeitersparnis gegenüber patientenspezifischen Resektionsschablonen zu erzwingen. Dies wurde durch die erfindungsgemäße Ausgestaltung erreicht.

Zusätzlich wurde zu den Resektionsschablonen auch eine speziell auf diese Technik abgestimmte Plattensystemausgestaltung zur Transplantatfixierung erarbeitet. Entgegen dem aktuellen Stand der Wissenschaft, nachdem zur Überbrückung eines Defekts oder zur Fixierung des Transplantats Rekonstruktionsplatten eingesetzt werden, werden nun wesentlich dünnere Miniplatten mit einer Profilstärke von 1 mm (1,0 mm) eingesetzt, da diese entsprechend den gewonnenen Erfahrungen einen deutlichen Vorteil bei der Materialentfernung bringen.

Platten, wie etwa Rekonstruktionsplatten (größer 1,0 mm dick, bis ca. 3,0 mm) oder Miniplatten (kleiner gleich 1,0 mm dick), die fachwerkartig ausgebildet sein können, werden auf die durchschnittliche Form des Unterkiefers angepasst bzw. auf das nach dem Sägeschnitt der Resektionsschablone entstehende Fibula-Transplantat vorgeformt. Dadurch wird eine hohe Passgenauigkeit erreicht und die Transplantatfixierung deutlich erleichtert.

Durchaus bedeutsam ist, dass die Unterkieferresektionsschablone auf die durchschnittliche Form des Unterkiefers angepasst ist. Die Resektionsschablone ist dabei so flexibel wie möglich in ihrer Einstellung, um eine Vielzahl an unterschiedlichen Resektionsmustern, in jedem Fall aber die folgenden Fälle, abzudecken: Drei-Segmentresektion (rechte Unterkieferkörper-Vordersegment-linker Unterkieferkörper), Zwei-Segmentresektion (rechter Unterkieferkörper inkl. vorderem Segment), Zwei-Segmentresektion (linker Unterkieferkörper inkl. vorderem Segment) und Ein-Segmentresektion. Auf diese Weise sind ca. 80 % aller theoretisch möglichen Resektionen abgedeckt.

Im hinteren Bereich der Resektionsschablone wird eine Längenanpassung möglich gemacht, die es erlaubt, den Sägeschlitz je nach Ausmaß des Defekts zu verschieben. Der flexible Sägeschlitz wird mit einer basal angebrachten Feststellschraube arretiert. Die Feststellschraube ist vorzugsweise nicht abnehmbar, um das Handling zu erleichtern und die Unfall- / Verlustgefahr zu verringern. Allerdings ist es in puncto Aufbereitung der Resektionsschablone vorteilhaft, wenn die Einzelteile leicht demontierbar sind und/oder entstehende Spalte so groß sind, das bei einer Desinfektion genutztes Medium einfach und wirkungsvoll reinigend wirken kann.

Zur Durchführung der Resektion wird eine oszillierende Säge verwendet. Es ist angedacht, den Sägeschlitz ca. 1 mm breit (+/- 0,1 bis 0,2 mm), und nach unten und/oder oben offen zu gestalten. Eine seitliche Führung des Sägeblatts ist in jedem Fall vorgegeben.

Die Resektionsschablone soll in erster Linie am Stück an den Unterkiefer angebracht werden können. Zusätzlich hierzu wird ergo dann ermöglicht, dass auch nur einzelne Segmente an den Unterkiefer angelegt werden. Über einen Steckmechanismus oder eine ähnliche Verbindungskonstellation werden das vordere Segment inklusive der rechten Seite bzw. das vordere Segment inklusive der linken Seite vom Rest abtrennbar gehalten.

Integrierte Bohrungen ermöglichen eine Fixierung der Schablone mittels Standardschrauben, die bspw. einen Durchmesser von 2,0 mm aufweisen, am Unterkiefer.

Eine angebrachte Mittellinienmarkierung im vorderen Segment wirkt sich vorteilhaft auf die Orientierung und genaue Ausrichtung der Resektionsschablone am Unterkiefer aus. Eine exakte Positionierung wird erleichtert.

Alle abnehmbaren Komponenten der Resektionsschablone werden zusätzlich Seiten-Markierungen erhalten, wie z.B. "R" für Rechts und "L" für Links. Eine Tumorentfernung bzw. Resektion des Unterkiefers erfolgt nach anatomischen Regionen (rechter Unterkieferkörper, Symphyse, linker Unterkieferkörper), d.h., wenn sich der Tumor bspw. mittig im Unterkieferkörper befindet, wird bis zum Unterkieferwinkel und zum Symphysenbereich resektiert.

Vorteilhafte Ausführungsformen sind in den Unteransprüchen beansprucht und werden nachfolgend näher erläutert.

So ist es von Vorteil, wenn die Positionierhilfe als eine Kimme-Korn-Kombination, eine Kerbe, eine Bohrung, ein Prisma, eine Farbmarkierung, ein Stift, eine Schraube oder eine Navigationspositionierung ausgebildet ist und/oder als eine Positionierflosse zum in Kontakt gelangen mit einem Abschnitt des Unterkieferknochens, etwa dem Symphysensegment, vorbereitete Positionierflosse ausgebildet ist.

Auch ist es von Vorteil, wenn die Positionierhilfe bzw. die Positionierflosse vom Zentralbauteil quer, vorzugsweise orthogonal absteht. Die Ausrichtung der Unterkieferresektionsschablone wird dadurch erleichtert.

Zweckmäßig ist es, dass das Zentralbauteil als Rahmen ausgebildet ist. Die Sicht auf den Unterkiefer wird dadurch verbessert.

Die Handhabbarkeit wird verbessert, weil der Rahmen zwei Horizontalstege besitzt, die vorzugsweise an deren distalen Enden über die beiden Sägeblatt-Führabschnitte miteinander verbunden sind.

Ein besonderes Ausführungsbeispiel ist dadurch gekennzeichnet, dass die beiden Trennwerkzeugführabschnitte / Sägeblattführabschnitte und die beiden Horizontalstege zumindest in einer Frontalprojektion eine rechteckige Form besitzen. Der Zusammenbau der Einzelbauteile wird dann erleichtert.

Es ist auch von Vorteil, wenn die beiden Horizontalstege und die beiden Trennwerkzeugführabschnitte / Sägeblattführabschnitte als ein einheitliches und/oder einstückiges / integrales und/oder einmaterialiges Bauteil ausgeformt sind oder zumindest einer der Trennwerkzeugführabschnitte / Sägeblattführabschnitte oder beide Trennwerkzeugführabschnitte / Sägeblattführabschnitte jeweils ein Bestandteil eines von den Horizontalstegen lösbaren / verschieblichen / separaten Verschiebebauteils ist.

Wenn zumindest einer der Trennwerkzeugführabschnitte / Sägeblattführabschnitte einen Sägeschlitz besitzt, der zum Führen eines Knochentrennwerkzeuges, wie eines Sägeblattes / einer Kreissäge, dimensioniert ist, so wird ein Verlaufen oder Verkanten des Knochentrennwerkzeuges verhindert.

Eine besonders gut wirkende Führung wird dadurch erreicht, wenn beidseitig des Sägeschlitzes eine zu diesem parallele Knochentrennwerkzeuganlagefläche ausgebildet ist.

Dabei ist es von Vorteil, wenn der Sägeschlitz vollständig durch das ihn ausbildende Material hindurch ragt und zusätzlich in Verlängerung seiner Längserstreckung einseitig oder zweiseitig offen ausgestaltet ist.

Besonders bruchresistent ist die Unterkieferresektionsschablone dann, wenn das Sägeschlitz beherbergende Material blockartig, vorzugsweise mit zueinander (nahezu) orthogonalen Oberflächen, ausgestattet ist und/oder einer der Horizontalstege rechteckbalkenartig ausgebildet ist, wobei die Horizontalstege bspw. geometrisch identisch ausgeformt sind.

Es ist von Vorteil, dass eine Mittellinienmarkierung an einer Vorder- und/oder Oberseite nur eines Horizontalsteges oder beider Horizontalstege vorhanden ist.

Wenn die Positionierflosse von einem der beiden Horizontalstege, vorzugsweise nur dem unteren der beiden Horizontalstege senkrecht und vorzugsweise auch senkrecht zu den beiden Sägeschlitzen absteht, etwa als integraler Bestandteil des Horizontalsteges, so kann die Unterkieferresektionsschablone gut von unten angebracht werden.

Es hat sich bewährt, wenn von einem oder jedem der beiden Trennwerkzeugführabschnitte / Sägeblattführabschnitte auf der zentralbauteilabgewandten Seite ein Erweiterungsbauteil anschließt. Der zu behandelnde Raum kann dann erweitert werden.

Dabei ist es hilfreich, wenn das Erweiterungsbauteil als ein integraler Bestandteil des Zentralbauteils ausgebildet ist oder als ein lösbar an einer Verbindungsstelle ankoppelbares und separates Erweiterungsbauteil ausgebildet ist.

Ein vorteilhaftes Ausführungsbeispiel ist auch dadurch gekennzeichnet, dass die Verbindungsstelle zwei zueinander passsende form- und/oder kraftschlüssig zusammenwirkende Koppelgeometrien einsetzt.

Gerade wenn die eine Koppelgeometrie einen Vorsprung ausformt und die andere Koppelgeometrie eine darauf abgestimmte Ausnehmung ausformt, lässt sich ein schnelles Zusammenstecken der Einzelbauteile bewirken.

Dabei hat es sich bewährt, wenn die Koppelgeometrien schwalbenschwanzartig zusammenwirken.

Ein vorteilhaftes Ausführungsbeispiel ist auch dadurch gekennzeichnet, dass die Ausnehmung als an einem Teilbereich entlang ihrer Längsachse offenes Sackloch ausgebildet ist.

Wenn ein Boden des Sackloches einen Anschlag für den Vorsprung definiert, so wird selbst in stressigen Situationen ein präziser Zusammenbau der Einzelteile der Unterkieferschablone erleichtert.

Die Resektion ist auch besonders gut durchführbar, wenn eine durch die Sägeschlitze verlaufende Ebene, bei Messung auf einer Unterkiefer zugewandten Seite, einen Winkel von ca. 10° bis ca. 20°, vorzugsweise ca. 12° +/- 5° zur oberen und unteren Horizontalstrebe einnimmt.

Es ist von Vorteil, wenn das Erweiterungsbauteil einen Rundkörper besitzt, von dem ein Zusatztrennwerkzeugführabschnitt / Zusatzsägeblattführabschnitt distal, d.h. an einem freien Ende, absteht, ergo auf einer verbindungsstellenfernen Seite vorhanden ist.

Es hat sich bewährt, wenn im Grundkörper zumindest ein Durchgangsloch zum Aufnehmen einer Knochenschraube vorhanden ist. Ein Verrutschen der Unterkieferschablone während der Resektion, oder davor oder danach, wird verhindert.

Um ein gutes Heranziehen beim Einschrauben der Knochenschraube der Resektionsschablone an dem Unterkiefer zu erzwingen, ist es von Vorteil, wenn das Durchgangsloch zu der Oberfläche des Grundkörpers schräg verläuft, bspw. angestellt ist.

Um auch ein Verschwenken zu vermeiden, ist es dabei von Vorteil, wenn zwei Durchgangslöcher parallel zur Längsachse des Erweiterungsbauteils, vorteilsweise zu einer Außenkante des Erweiterungsbauteils versetzt, angeordnet sind.

Dabei hat es sich bewährt, wenn die Durchgangslöcher zur unteren Außenkante hin versetzt sind, Vorzugsweise sind die Durchgangslöcher aber im unteren Drittel des Erweiterungsbauteils angeordnet.

Genauer gesagt ist es also zweckmäßig, wenn die Durchgangslöcher von "vorne" nach "hinten" ansteigend verlaufen. "Vorne" ist dabei jener Bereich, der vom Patienten außerhalb angeordnet ist, wohingegen "hinten" dann auf der patienteninneren Seite angeordnet ist. "Unten" ist dabei durch die Schwerkraft bestimmt, genauso wie "oben".

Wenn eine Zentralachse eines Durchgangsloches zur vorderen oder hinteren, im Wesentlichen vertikal ausgerichteten Oberfläche, einen Winkel von ca. 20° +/- 5° einnimmt, so zieht sich beim Anschrauben der befestigenden Schrauben die Schablone gut passend an den Knochen heran.

Es hat sich bewährt, wenn der Zusatztrennwerkzeugführabschnitt / Zusatzsägeblattführabschnitt vom Grundkörper entfernbar ist, etwa über einen Verstellmechanismus. Die Flexibilität des Einsatzes bzw. die Einsatzmöglichkeiten werden dadurch erhöht.

Wenn der Zusatzsägeblattabschnitt einen Durchgangsschlitz zwischen zwei Führungsflächen besitzt, so kann auch dort die Präzision des vorzunehmenden Schnittes erhöht werden. Dabei ist vorzugsweise ein oberes oder unteres Ende des Durchgangsschlitzes offen zu lassen.

Ebenfalls hat es sich bewährt, wenn der Zusatzsägeblattabschnitt ein von einem Block abstehenden Stab besitzt, der in einem bspw. offenen Kanal oder einer Rinne verstellbar gehalten ist.

Es ist von Vorteil, wenn der Stab im Kanal über ein Arretiermittel, wie eine Schraube, festlegbar ist.

Auch ist es von Vorteil, wenn am Erweiterungsbauteil ein Unterkieferauflagebock vorhanden ist.

Es ist wünschenswert, wenn der Unterkieferauflagebock dann (nahezu) senkrecht von der hinteren Oberfläche des Grundkörpers oder des Zusatzsägeblattabschnittes absteht.

Wenn im Unterkieferbock ein Langloch ausgebildet ist, dessen längere Querachse durch die Längsrichtung des Unterkieferloches bestimmt ist, so wird ein einfaches Positionieren bei doch vorliegender Exaktheit möglich.

Von Vorteil ist es auch, wenn zwei Erweiterungsbauteile vorhanden sind, die zu einer Mittelebene spiegelsymmetrisch zueinander sind, wobei die Mittelebene jene Ebene ist, in der der Grat liegt und auf der die Horizontalstreben senkrecht stehen.

Ein vorteilhaftes Ausführungsbeispiel ist ferner dadurch gekennzeichnet, dass das Zentralbauteil mit seinen an beiden Enden abstehenden Erweiterungsbauteilen eine solche gebogene Form aufweist, die der Außenkontur eines durchschnittlichen menschlichen Unterkiefers (äquidistant) folgt

Es soll auch erwähnt werden, dass der Stab auf zumindest einer Oberfläche, etwa der Frontfläche, eine quer zur Längsrichtung des Stabes ausgerichtete Riffelung oder Rasterung besitzt.

Es ist von Vorteil, wenn die Unterkieferschablone aus Metall, etwa einer Titanlegierung, oder Kunststoff, etwa einem Polymer (vollständig) aufgebaut ist. Hier sind bspw. Edelstahl, Titanlegierungen und Kunststoffe, etwa ABS-Kunststoffe denkbar.

Die Erfindung betrifft in einem Nebenaspekt auch eine Fibula-Knochenmaterialentnahme- und -verbringschablone mit einem Mittelteil, das einen Zentralkörper besitzt, an dessen Enden je ein Knochentrennwerkzeugführabschnitt vorhanden ist. Eine solche Fibula-Knochenmaterialentnahme- und -verbringschablone wird dadurch verbessert, wenn zumindest einer der Knochentrennwerkzeugführabschnitte, vorzugsweise beide Knochentrennwerkzeugführabschnitte, vom Mittelteil entfernbar oder heranschiebbar gelagert ist/sind.

Diese Fibula-Knochenmaterialentnahme- und -verbringschablone kann nur zusätzlich zu der Unterkieferresektionsschablone beansprucht werden, also mit den Merkmalen des Patentanspruchs 1.

Eine solche schon wesentlich verbesserte Fibula-Knochenmaterialentnahme- und - verbringschablone kann auch noch weiter verbessert werden. Diese weiterführenden Verbesserungen werden nachfolgend näher erläutert.

So ist es von Vorteil, wenn der Knochentrennwerkzeugführabschnitt einen zwischen zwei Vertikalflächen ausgebildeten Führungsschlitz besitzt, der auf dessen Vorder- und Rückseite offen ist. Auf diese Weise wird bei der Entnahme von Knochen aus dem Wadenbein ein Verlaufen des Knochentrennwerkzeuges verhindert. Eine präzise Entnahme wird dadurch sichergestellt.

Für das Handling zuträglich ist es, wenn der Führungsschlitz auf dessen Unterseite oder Oberseite offen ausgestaltet ist.

Die Einstellbarkeit und Flexibilität im Einsatz / während der Operation wird verbessert, wenn von dem zum Mittelteil separaten Knochentrennwerkzeugführabschnitt ein Balken absteht, der in einer ihn umfassenden Führungsbahn entlang ihrer Längsrichtung verschieblich gelagert ist.

Zweckmäßig ist es auch, wenn in die Führungsbahn eine Fixierschraube ragt, die zum Befestigen des Balkens ausgelegt ist. Intraoperativ kann dann eine einfache Veränderung durchgeführt werden oder zumindest präoperativ vorbereitet sein.

Es hat sich bewährt, wenn etwa in der Mitte des Mittelteils eine Bügelaufnahmevorrichtung, wie eine Klammer, vorhanden ist, in die ein Entnahmehilfsbügel einsteckbar ist oder eingesteckt ist. Die einzelnen Bestandteile der Fibula-Knochenmaterialentnahme- und -verbringschablone können dann zueinander im Raum unveränderbar festgelegt werden.

Dabei ist es von Vorteil, wenn in der Bügelaufnahmevorrichtung ein Loch, wie ein Sackloch oder ein Durchgangsloch vorhanden ist, das zum arretierenden Aufnehmen eines entnahmehilfsbügelfesten Federabschnittes vorbereitet ist. Ein schnelles Einsetzen und Entfernen des Entnahmehilfsbügels wird dann möglich.

Es hat sich auch bewährt, wenn beiderseits der Klammer ein Aufnahmeloch für eine Knochenschraube vorhanden ist.

Für eine gute Anbringbarkeit zuträglich ist es, wenn das Aufnahmeloch eine Symmetrieachse besitzt, die quer, vorzugsweise senkrecht zu einer den Zentralkörper aufnehmenden Zentralebene ausgerichtet ist.

Es ist auch zuträglich für einen flexiblen operativen Einsatz, wenn auf einer oder jeder der beiden Knochentrennwerkzeugführabschnitte ein Ergänzungsbauteil auf der zentralkörperabgewandten Seite vorhanden ist. Ferner ist es von Vorteil, wenn an dem distalen Ende des Ergänzungsbauteils jeweils ein weiterer Knochentrennwerkzeugführabschnitt verschiebbar und vom Ergänzungsbauteil entfernbar angeordnet ist.

Ein vorteilhaftes Ausführungsbeispiel ist auch dadurch gekennzeichnet, dass die Knochentrennwerkzeugführabschnitte des Ergänzungsbauteils den Knochentrennwerkzeugführabschnitten des Zentralkörpers identisch oder zumindest ähnlich sind.

Ein vorteilhaftes Ausführungsbeispiel kann auch so ausgestaltet sein, dass an der am distalen freien Ende des Ergänzungsbauteils vorhandenen Knochentrennwerkzeugführabschnittsausgestaltung zwei Führungsstege vorhanden sind, die in einem gemeinsamen Führungsblock in eigenen Öffnungen geführt sind.

Wenn in dem Führungsblock eine, zwei oder mehr nur den oberen Führungssteg berührende Feststellschraube(n) eingesetzt ist/sind, so kann ein gut zugänglicher Bereich für das Arretieren und Lösen der Arretierung gewählt / genutzt werden. Die Feststellschraube ist vorzugsweise nicht abnehmbar.

Es hat sich auch bewährt, wenn an einem oder beiden Führungsstegen eine Rasterung oder Kerbung vorhanden ist, insbesondere auf der Frontseite mit Rippen, die quer, vorzugsweise orthogonal zur Längsrichtung eines Führungssteges oder beider Führungsstege verlaufen. Eine haptische Rückkoppelung an den Operateur ist dann relativ einfach realisierbar.

Es ist von Vorteil, wenn an dem Ergänzungsbauteil eine Bügelaufnahmevorrichtung zum arretierenden Aufnehmen des Entnahmehilfsbügels vorhanden ist.

Ein vorteilhaftes Ausführungsbeispiel ist auch dadurch gekennzeichnet, dass die Bügelaufnahmevorrichtung des Ergänzungsbauteils identisch oder zumindest ähnlich zur Bügelaufnahmevorrichtung des Zentralkörpers ausgebildet ist.

Es hat sich auch bewährt, wenn die beiden Ergänzungsbauteile zu einer mittig durch den Zentralkörper und von dieser senkrecht durchdrungenen Symmetrieebene spiegelsymmetrisch ausgebildet sind.

Es ist von Vorteil, wenn der Entnahmehilfsbügel zumindest zwei oder vier (90°-) Stufen besitzt. Eine Deformation von Weichgewebe wird dadurch vermieden.

Es ist für die Erfindung zuträglich, wenn die Rückseite der Schablone zum Kontaktieren eines (menschlichen) Wadenbeins vorbereitet ist.

Es ist wünschenswert, wenn die beiden Führungsschlitze am Zentralkörper einen ca. 60°-Winkel +/- 5° und die beiden Führungsschlitze des Ergänzungsbauteils in Richtung der Rückseite einen spitzen Winkel einschließen, etwa einen ca. 72°-Winkel +/- 5°.

Von Vorteil ist es dabei, wenn die Bügelaufnahmevorrichtungen zum Aufnehmen eines starren / steifen / un-elastischen / formstabil (ähnlich einem Stahlbauteil) Implantierhilfsbügels vorbereitet sind. Dadurch können die einzelnen Knochenstücke positionstreu verlagert werden.

Wenn der Implantierhilfsbügel dem Entnahmehilfsbügel identisch oder ähnlich ist, sich aber in der dem Ergänzungsbauteil und dem Zentralkörper relativ zueinander aufgezwungenen Lage unterscheidet, ergo eine geometrisch an den Anbindungsstellen andere Ausgestaltung hat, so lässt sich ein Überführen der Knochen, die dem Wadenbein entnommen wurden, effizient zum Unterkiefer mit seinen dortigen Lücken realisieren.

Ferner ist es von Vorteil, wenn der Entnahmehilfsbügel das Ergänzungsbauteil und den Zentralkörper in eine gemeinsame Ebene zwingt, jedoch der Implantierhilfsbügel das Ergänzungsbauteil und den Zentralkörper in eine U-Form und/oder eine unterkieferkonturkonforme Ausrichtung zwingt.

Die Fibula-Knochenmaterialentnahme- und -verbringschablone kann auch als Fibula-Resektionsschablone bezeichnet werden und ist auf die durchschnittliche Form der Fibula angepasst. Sie ist also nicht patientenspezifisch sondern an den durchschnittlichen Patienten angepasst.

Die einzelnen Segmente und Längen sind auf die Unterkieferresektionsschablone abgestimmt. Die Skalen der beiden Schablonen sollten einheitlich gestaltet und aufeinander abgestimmt sein. Eine Berücksichtigung der präoperativen Planung hat jedoch nicht zwingend zu erfolgen.

Mit einem abnehmbaren Bügel werden die einzelnen Segmente miteinander verbunden. Der Bügel soll sowohl von oben als auch von unten anbringbar sein, um die Schablone für die rechte und linke Fibula gleichermaßen zu verwenden. Darüber hinaus soll der Bügel mit einer kleinen Stufe versehen werden, um weiter nach vorne abzustehen, da dieser Bereich häufig durch Weichgewebe behindert wird.

Die Stellschrauben zur Arretierung der flexiblen Wegeschlitze sollen vorzugsweise orthogonal zur Schablone angebracht werden.

Zur Durchführung der Resektion soll eine oszillierende Säge verwendet werden. Es ist angedacht, den Sägeschlitz ungefähr 1,0 mm breit nach unten oder oben oder beidseitig offen oder geschlossen zu gestalten. Eine seitliche Führung des Sägeblattes sollte jedoch in allen Fällen stattfinden.

Die Fixierung der Schablone an der Fibula erfolgt mit Standardschrauben, die einen Außendurchmesser von ca. 2,0 mm besitzen. In jedem Segment befinden sich zwei Bohrungen für Fixierungen.

Die Schablone sollte bestmöglich eine Fixierung der resezierten Fibula-Segmente mittels Implantate von vorne ermöglichen. Gegenwärtig wird das rechte und linke Segment jeweils durch eine im Winkel von ca. 120° angebogene Platte mit dem vorderen Segment verbunden. Die Platte wird von oben angebracht.

Miniplatten mit einem Profil von 1,0 mm, die eine einfache Transplantatfixierung ermöglichen, und anhand der durchschnittlichen Form des Unterkiefers bzw. Fibula-Transplantat bis zu dreidimensional vorgeformt sind, haben sich bewährt. Die Kontur des Durchschnittsunterkiefers sowie der Fibula werden auf Basis repräsentativer Datensätze generiert.

Die Platten müssen in unterschiedlichen Formen und Ausführungen zur Verfügung gestellt werden. Ziel ist es, mit so wenig wie möglich Platten auszukommen. Die exakten Varianten müssen zusammen dann noch ermittelt werden. Denkbar sind jedoch Vier-Lochplatten mit Steg und/oder Sechs-Lochplatten mit Steg. Die Platten sollten über multidirektionale winkelstabile Plattenlöcher verfügen, um sowohl verblockend versorgen zu können, als auch mit Standardschrauben zusammen zu wirken. Je nach Notwendigkeit können spezielle Instrumente zur Fixierung der Platte, bspw. Schraubendreher eingesetzt werden.

Die Erfindung wird nachfolgend mit Hilfe einer Zeichnung näher erläutert, in der unterschiedliche Ausführungsformen dargestellt sind. Es zeigen:
- Fig. 1: eine erfindungsgemäße Unterkieferresektionsschablone in einer perspektivischen Darstellung,
- Fign. 2 bis 5: die Unterkieferresektionsschablone der Fig. 1 in Anlage an einem Unterkiefer in unterschiedlichen Ansichten (von vorne, von der rechten Seite, von der linken Seite und von oben), jeweils leicht perspektivisch,
- Fign. 6 bis 9: weitere Darstellungen mit unterschiedlichen Unterkiefern ähnlich der Darstellungen der Fign. 2 bis 5,
- Fig. 10: eine perspektivische Darstellung einer erfindungsgemäßen FibulaKnochenmaterialentnahme- und -verbringschablone,
- Fig. 11: die Fibula-Knochenmaterialentnahme- und -verbringschablone von hinten, d.h. von der Unterkieferseite,
- Fig. 12: eine weitere FibulaKnochenmaterialentnahme- und -verbringschablone mit niedrigem Entnahmehilfsbügel,
- Fig. 13: eine Ansicht von oben auf die Fibula-Knochenmaterialentnahme- und -verbringschablone aus Fig. 12 in Anlage an eine Fibula mit bereits durchgeführten Schnitten,
- Fig. 14: die Fibula-Knochenmaterialentnahme- und -verbringschablone aus Fig. 13 mit entfernten Restknochenabschnitten, wobei die zu transplantierenden Knochenabschnitte an der Schablone befestigt sind,
- Fign. 15 bis 17: eine zweite Ausführungsform einer Fibula-Knochenmaterialentnahmeund -verbringschablone, mit einem höheren Entnahmehilfsbügel und mehrere Schlitze gemeinsam aufnehmenden Blöcken, wobei die Fig. 15 der Darstellung der Fig. 12 entspricht und die Fig. 16 einer Darstellung der Fig. 13 entspricht sowie die Fig. 17 einer Darstellung der Fig. 14 entspricht,
- Fig. 18: die Fibula-Knochenmaterialentnahme- und -verbringschablone gemäß der Erfindung in einer Verbringposition, bei der die zu transplantierenden Knochenstücke in die Form des zu ersetzenden oder zu reparierenden Unterkieferknochens verbracht sind,
- Fign. 19 bis 20: eine weitere Darstellung der erfindungsgemäßen Fibula-Knochenmaterialentnahme- und -verbringschablone in Kontakt mit einem Wadenbein / einer Fibula in einer Ansicht von vorne (Fig. 19), von der Seite (Fig. 20) und in einer perspektivischen Ansicht (Fig. 21),
- Fign. 22 bis 24: eine weitere Darstellung des am Fibula-Knochen angebrachten Fibula-Knochenmaterialentnahme- und -verbringschablone, in mehreren den Fign. 19 bis 21 entsprechenden Darstellungsarten, wobei die zu entnehmenden Knochen dargestellt sind,
- Fign. 25 bis 27: die entnommenen Knochenstücke an der angebrachten FibulaKnochenmaterialentnahme- und -verbringschablone in zu den Fign. 22 bis 24 vergleichbaren Darstellungsarten,
- Fign. 28 bis 30: den zum ersten Entnahmehilfsbügel geometrisch veränderten Implantierhilfsbügels, mittels dessen die entnommenen Knochenstücke in eine unterkieferähnliche Form gebracht werden und somit transplantierfähig vorbereitet werden,
- Fign. 31 bis 33: die in Position gebrachten Fibula-Knochenstücke im an den Restunterkiefer eingesetzten Zustand in einer Ansicht von oben, von vorne und einer perspektivischen Darstellung,
- Fign. 34 bis 38: die transplantierten Knochen im Unterkieferbereich in unterschiedlichen räumlichen Darstellungen (von oben, von vorne, perspektivisch von vorne, perspektivisch von der Seite rechts und perspektivisch von der Seite links).

Die Figuren sind lediglich schematischer Natur und dienen nur dem Verständnis der Erfindung. Die gleichen Elemente sind mit denselben Bezugszeichen versehen. Merkmale der einzelnen Ausführungsbeispiele können untereinander ausgetauscht werden.

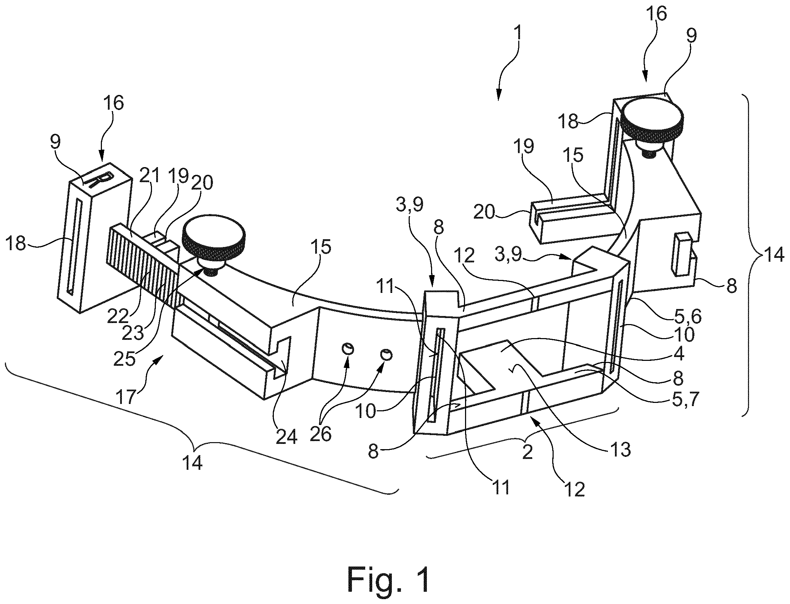

In Fig. 1 ist eine erfindungsgemäße Unterkieferresektionsschablone 1 dargestellt. Die Unterkieferresektionsschablone 1 besitzt ein Zentralteil / Zentralbauteil 2. Dieses Zentralbauteil 2 ist zum Verbrachtwerden an einem Symphysensegment eines Unterkiefers vorbereitet. Das Zentralbauteil 2 weist an beiden äußeren Enden, wobei die Enden die Längsachse definieren, zwei Trennwerkzeugführabschnitte / Sägeblattführabschnitte 3 auf. Zwischen den beiden Trennwerkzeugführabschnitten / Sägeblattführabschnitten 3 ist eine Positionierhilfe / Positionierflosse 4 vorhanden. Die Trennwerkzeugführabschnitte müssen nicht in Kontakt gelangen mit einem Trennwerkzeug, wohingegen die dazu vergleichbaren Sägeblattführabschnitte durchaus zum physischen in Kontaktgelangen mit einem Trennwerkzeug, wie einem Fräser, einem Sägeblatt oder einem anderen metallischen spanabhebenden Werkzeug. Die Positionierhilfe muss auch nicht zwingen in physischem Kontakt oder gar in Anschlag mit dem Knochen gelangen, sondern kann beispielsweise auch nur optische Mittel zur Ausrichtung nutzen. Bei Ausgestaltung der Positionierhilfe als Positionierflosse ist der ein physisches in Kontaktgelangen mit dem Knochen aber wünschenswert.

Die Positionierflosse 4 steht hier jedenfalls senkrecht auf einer Ebene durch das Zentralbauteil 2 in Richtung des Unterkiefers von einem horizontal Steg 5 ab. Es gibt einen oberen Horizontalsteg 6 und einen unteren Horizontalsteg 7. Die Positionierflosse 4 steht vom unteren Horizontalsteg 7 ab. Sie ist im hier vorliegenden Ausführungsbeispiel plattenartig ausgebildet, kann aber auch stiftartig ausgebildet sein, etwa mit kreisrundem, elliptischem oder polygonalem Querschnitt.

An distalen Enden 8 der Horizontalstege 5 sind die Sägeblattführabschnitte 3 nach Art von Blöcken 9 einstückig und einmaterialig angebracht. In den Blöcken 9 sind Sägeschlitze 10 vorhanden. Pro Sägeblattführabschnitt 3 ist ein Sägeschlitz 10 vorhanden. Der Sägeschlitz 10 befindet sich zwischen zwei parallel zueinander verlaufenden vertikal ausgerichteten Knochentrennwerkzeuganlageflächen 11.

Am unteren Horizontalsteg 7 vorne und/oder unten ist eine Markierung 12 als Mittellinienmarkierung ausgebildet, genauso wie am oberen Horizontalsteg 6 vorne und/oder oben.

Beiderseits des Zentralbauteils 2 steht jeweils ein Erweiterungsbauteil 14 ab. Die beiden Erweiterungsbauteile 14 sind hier als integrale Bestandteile des Zentralbauteils 2 auskonstruiert. Allerdings kann ein Erweiterungsbauteil 14 oder beide Erweiterungsbauteile 14 auch lösbar am Zentralbauteil 2, nämlich auf der Außenseite je eines Blockes 9 angekoppelt werden. Dazu sind dann entsprechende Verbindungsstellen mit Koppelgeometrien, wie Vorsprüngen und Ausnehmungen, etwa nach Art von Schwalbenschwanzkonfigurationen, bspw. unter Ausbildung eines Bodens und eines Anschlages gestaltbar.

Jedenfalls weist jedes Erweiterungsbauteil 14 einen Grundkörper 15 auf, an dessen Ende jeweils ein Zusatzsägeblattführabschnitt 16 vorhanden ist. Dabei ist ein Verstellmechanismus 17 eingesetzt, um eine Verschiebbarkeit des Zusatzsägeblattführabschnittes 16, nach Art eines weiteren Blockes vom Grundkörper 15 zu gewährleisten. Auch in den blockartigen Zusatzsägeblattführabschnitten 16 sind Durchgangsschlitze 18 vorhanden, die ähnlich oder identisch zu den Schlitzen / Sägeschlitzen 10 in den Sägeblattführabschnitten 3 ausgestattet sind.

Von den Enden des Grundkörpers 15, etwa im Bereich des Verstellmechanismus 17 oder aber von den Zusatzsägeblattführabschnitten 16 steht ein Unterkieferauflagebock 19 mit einem Langloch ab. Das Langloch ist nicht dargestellt. Im hier vorgestellten Ausführungsbeispiel wird eine Schlitzverlängerung 20 stattdessen genutzt. Das Langloch kann also die Schlitzverlängerung 20 ersetzen, die Längsachse des als Durchgangsloch ausgebildeten Langlochs ist vorzugweise in Richtung des Schlitzes der Schlitzverlängerung 20 ausgerichtet.

Der Verstellmechanismus weist einen Stab 21 auf, auf dessen Frontfläche / Frontoberfläche 22 eine Riffelung 23 vorhanden ist. Der Stab 21 greift in einen offen konstruierten Kanal 24 und wird durch ein als Feststellschraube ausgebildetes Arretiermittel 25 lösbar in Position gehalten. Es gibt in jedem Erweiterungsbauteil 14 zumindest zwei Durchgangslöcher 26 für die Befestigung der Unterkieferresektionsschablone 1 mittels Schrauben am hier nicht dargestellten Unterkiefer.

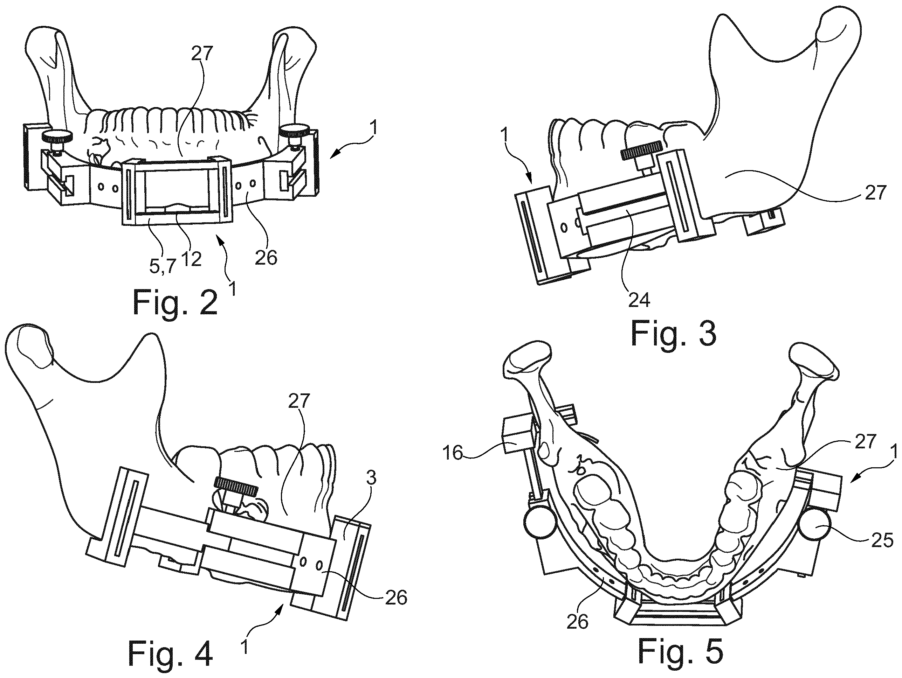

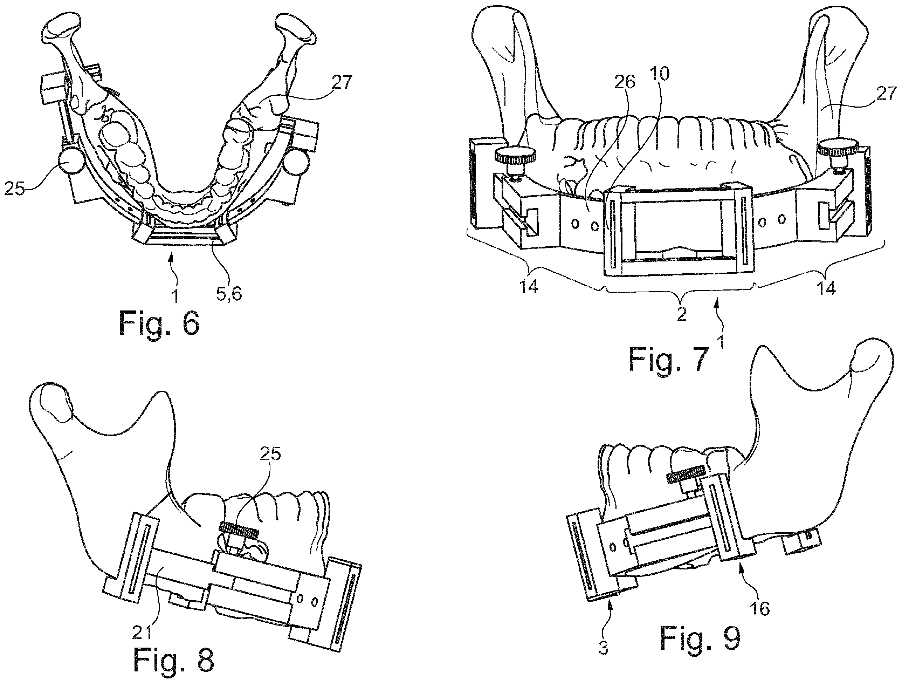

In den Fign. 2 bis 9 ist die erfindungsgemäße Unterkieferresektionsschablone in unterschiedlichen Positionen an einem Unterkiefer 27 dargestellt.

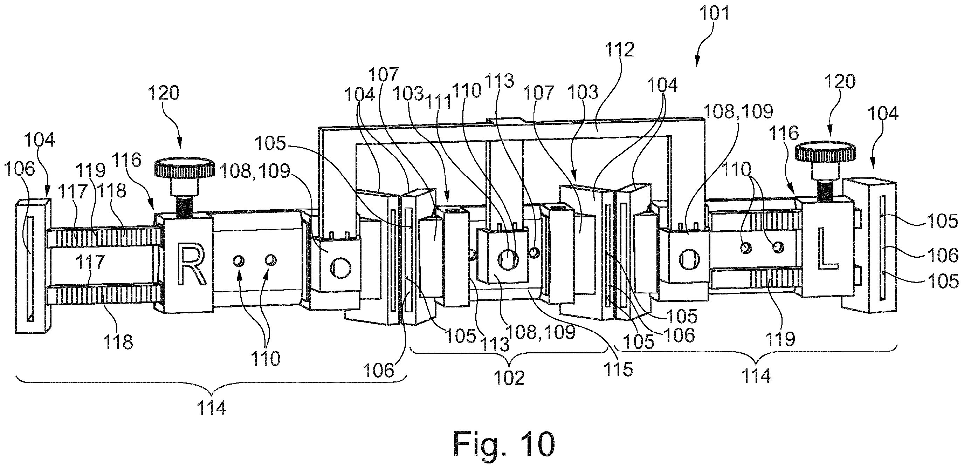

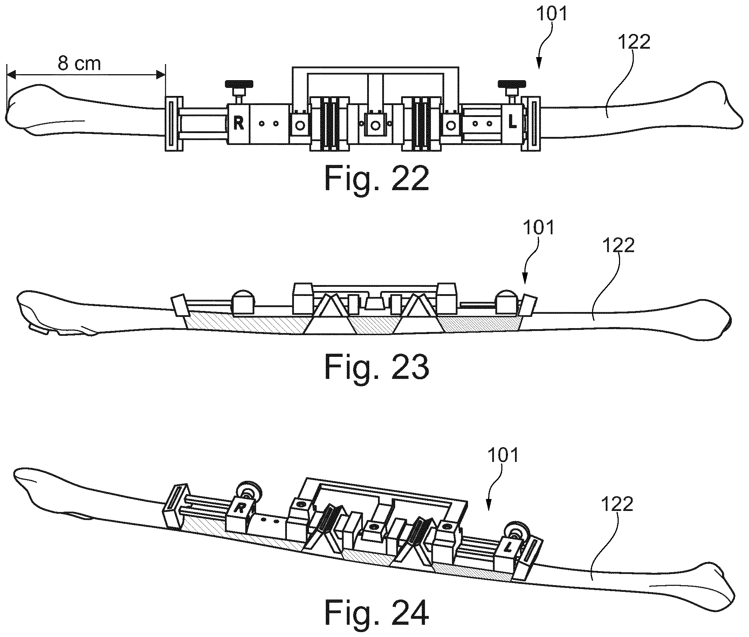

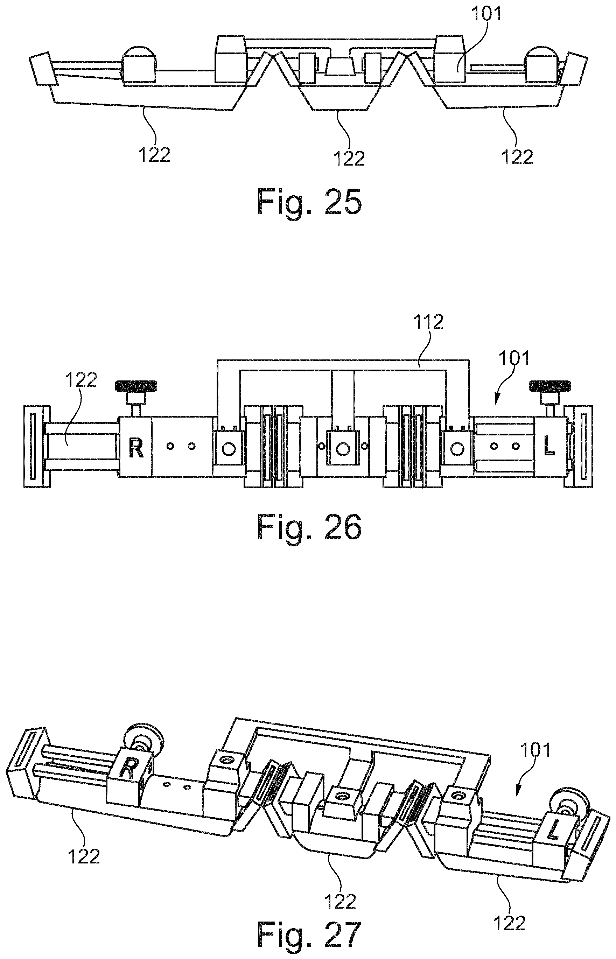

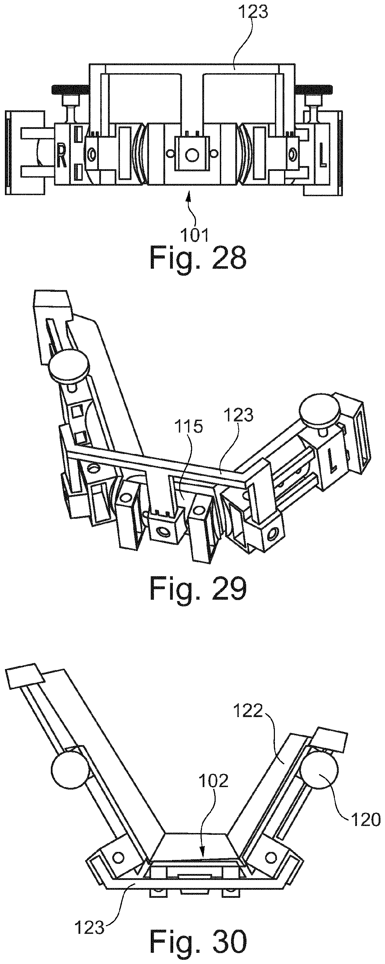

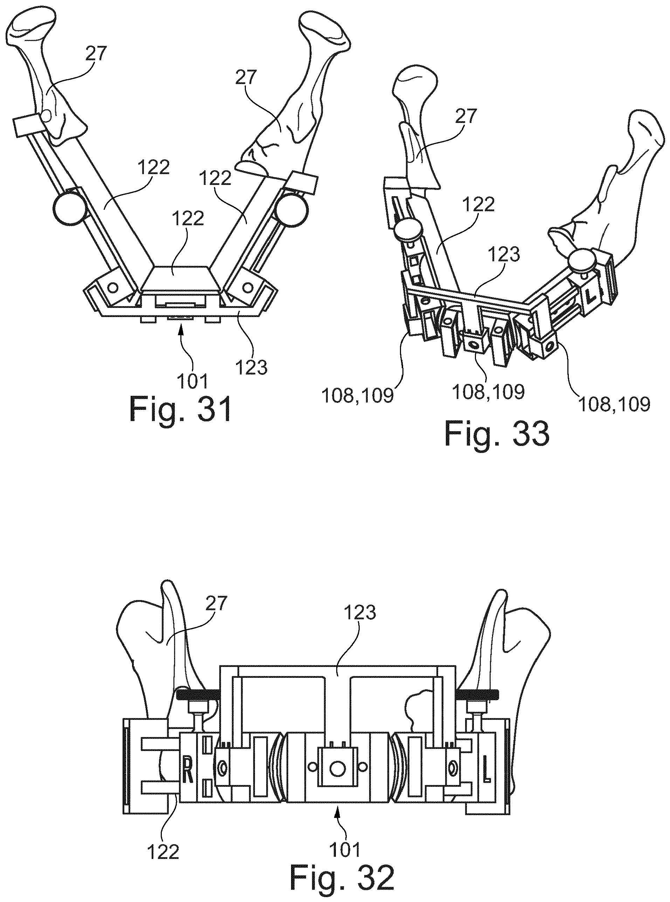

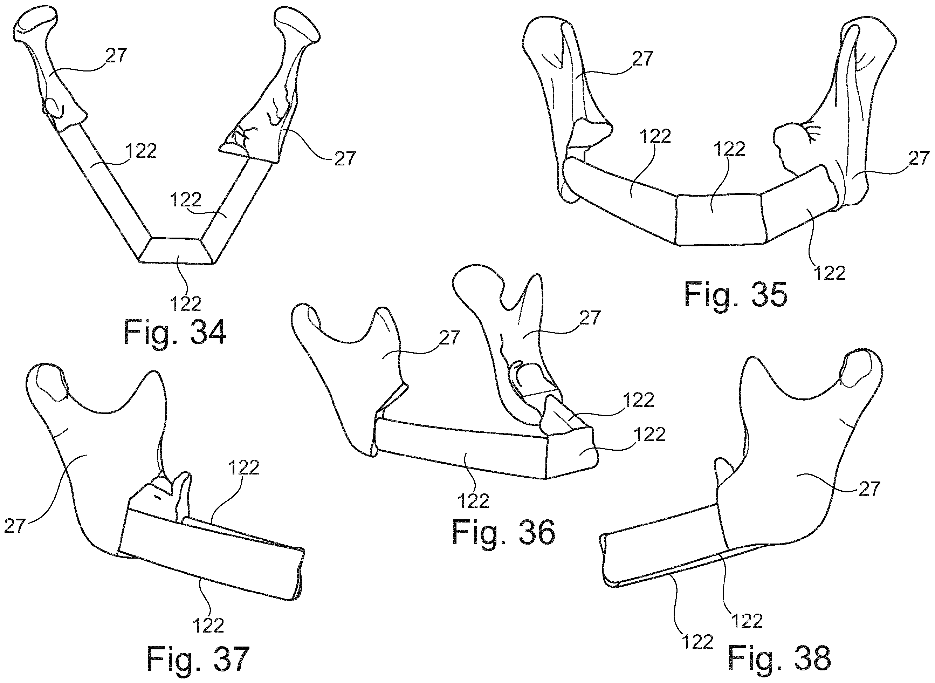

In den Fign. 10 bis 38 ist nun nachfolgend vermehrt der Augenmerk auf eine erfindungsgemäße Fibula-Knochenmaterialentnahme- und -verbringschablone bzw. den an einem Fibula-Knochen entnommenen Knochen und zu einem in den Unterkiefer zu transplantierenden Knochenstücken Augenmerk gelegt.

So ist in Fig. 10 eine erfindungsgemäße Fibula-Knochenmaterialentnahme- und - verbringschablone 101 dargestellt. Diese Fibula-Knochenmaterialentnahme- und - verbringschablone 101 weist ein Mittelteil 102 auf. An dessen distalen Enden 103 ist jeweils ein Knochentrennwerkzeugführabschnitt 104 vorhanden. Diese Knochentrennwerkzeugführabschnitte 104 können als Backen oder Blöcke ausgebildet sein und entweder einstückig mit dem Mittelteil bzw. eines Grundkörpers / Basiskörpers des Mittelteils 102 verbunden sein oder verschiebbar angekoppelt sein. Zumindest einer der Knochenwerkzeugführabschnitte 104 sollte aber wegebewegbar, bspw. klappbar und/oder entfernbar und heranschiebbar gelagert sein.

Jeder Knochentrennwerkzeugführabschnitt 104 weist zwischen zwei Vertikalflächen 105 einen Führungsschlitz 106 auf. Diese Führungsschlitze 106 sind auf der Vorder- und Rückseite offen / durchlässig ausgestaltet. Es ist jeder Führungsschlitz 106 bis auf die länglichen Öffnungen in der Vorder- und Rückseite vollständig von Material umgeben. Allerdings kann ein Führungsschlitz 106 unten und/oder oben offen sein.

Für eine Verschiebbarkeit eines Knochentrennwerkzeugführungsabschnitts 104 bietet sich das Vorhalten eines Balkens 107 an. Der Balken 107 ist in einer ihn umfassenden Führungsbahn verschieblich gelagert. Eine nicht dargestellte Fixierschraube kann dabei zum Befestigen des Balkens 107 eingesetzt sein.

In der Mitte des Mittelteils 102 befindet sich eine Bügelaufnahmevorrichtung 108. Die Bügelaufnahmevorrichtung 108 ist hier als Klammer 109 ausgebildet und weist ein Durchgangsloch 110 auf, in das ein konvexer Federabschnitt 111 eines Entnahmehilfsbügels 112 eingreift. Links und rechts der Klammer 109 befindet sich jeweils ein Aufnahmeloch 113, um eine Knochenschraube aufzunehmen, mittels derer ein Anbringen an einem Fibula-Knochen durchgeführt werden kann. Die Aufnahmelöcher 113 sind nach Art von Bohrungen ausgebildet.

Beiderseits des Mittelteils 102 schließt jeweils ein Ergänzungsbauteil 114 an. Zwischen dem Ergänzungsbauteil 114 ist somit ein Zentralkörper 115 des Mittelteils 102 angeordnet.

Jedes Ergänzungsbauteil 114 weist einen weiteren Knochentrennwerkzeugführabschnitt 104 auf. Jeder dieser Knochentrennwerkzeugführabschnitte 104 der Ergänzungsbauteile 114 ist dann über einen Verschiebemechanismus 116 verschieblich gehalten. Dabei werden jeweils zwei Führungsstege 117 eingesetzt, die beide auf ihrer Frontseite 118 Rippen, Kerben oder Rasten besitzen, um eine Rasterung 119 auszubilden. Es sind wiederum Bügelaufnahmevorrichtungen 108 nach Art von Klammern 109 vorhanden, in die Enden des Entnahmehilfsbügels 112 eingreifen. Die Verbindung zwischen dem Entnahmehilfsbügel 112 und den Klammern 109 ist ähnlich oder identisch wie schon vorher ausgeführt ausgebildet.

In jedem der Ergänzungsbauteile 114 sind ebenfalls Durchgangslöcher 110 vorhanden, um eine Befestigung mit Knochen über Schrauben zu ermöglichen. Der Entnahmehilfsbügel 112 ist abnehmbar zur Schablonenfixierung. Es sind Feststellschrauben 120 zur Fixierung des flexiblen Wegeschlitzes eingesetzt.

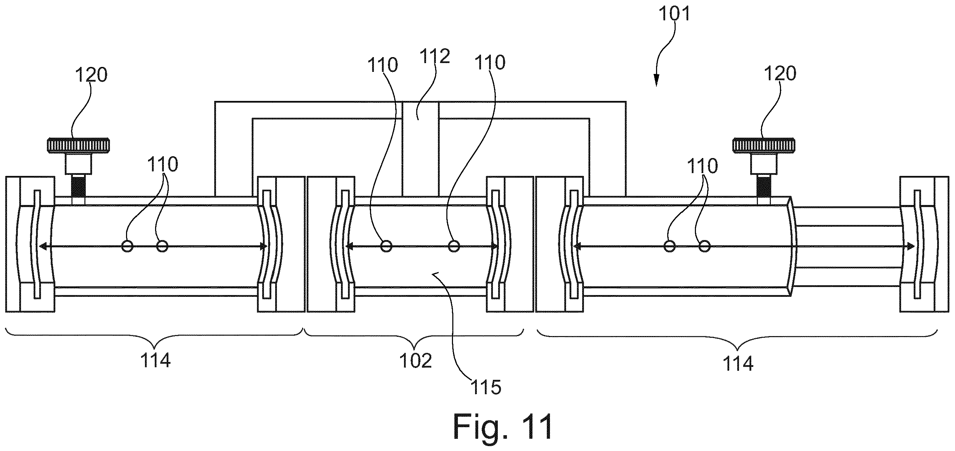

Während in der Fig. 10 die Fibula-Knochenmaterialentnahme- und -verbringschablone im Wesentlichen von vorne gezeigt ist, ist diese in der Fig. 11 im Wesentlichen von hinten, also vom Fibula-Knochen aus gesehen, dargestellt. Man sieht also die Rückseite. Die Länge der Ergänzungsbauteile 114 sollte möglichst zwischen 45,2 mm und 66,2 mm variierbar sein. Die Sequenzlänge des Mittelteils 102 sollte möglichst 30 mm betragen und kann fix sein.

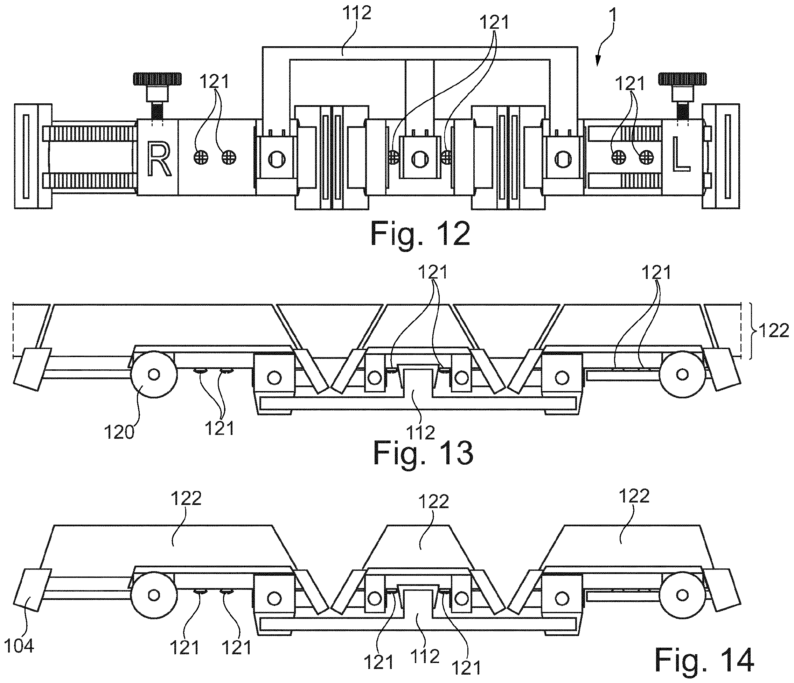

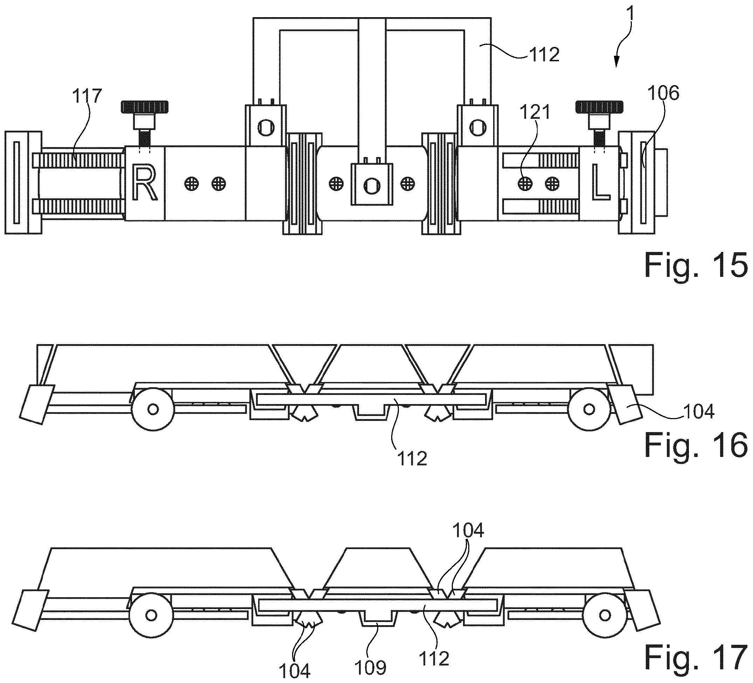

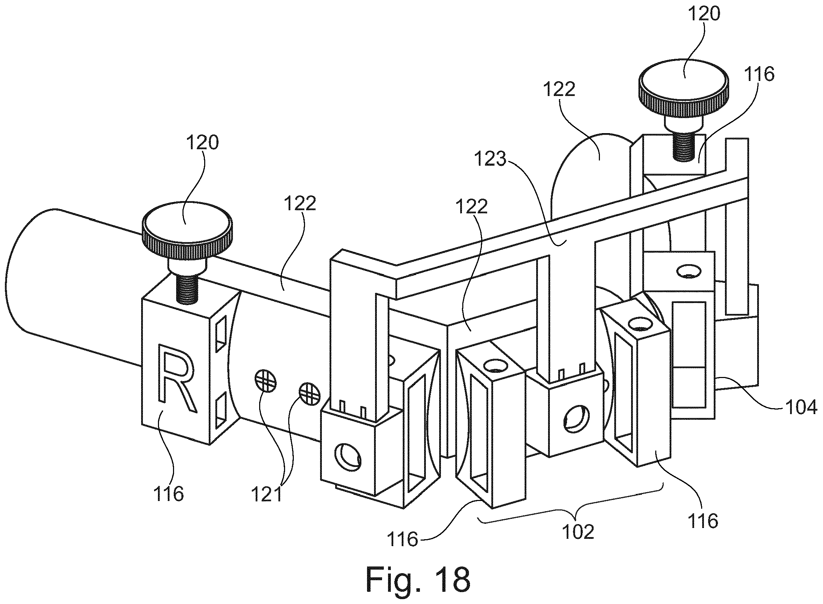

In den Fign. 12 und 15 sind bereits Knochenschrauben 121 eingesetzt, um, wie in den Fign. 13 und14 und 16 sowie 17 die Befestigung an Teilen eines Fibula-Knochens 122 zu bewerkstelligen. In Fig. 18 ist ein geometrisch vom Entnahmehilfsbügel 112 leicht veränderter Implantierhilfsbügel 123 eingesetzt. Die einzelnen Abschnitte des Fibula-Knochens 122 werden dann komplett neu angeordnet, ähnlich in der räumlichen Lage wie von der Unterkieferresektionsschablone 1 vordefiniert, vorzugsweise identisch. Die distal äußersten Knochentrennwerkzeugführabschnitte 104 sind dabei entfernt worden. Dies gilt auch für die Knochentrennwerkzeugführabschnitte 104 des Mittelteils 102. Die Sägeschlitze bzw. Führungsschlitze 106 wurden entfernt, um die Segmente "Stoß auf Stoß" setzen zu können.

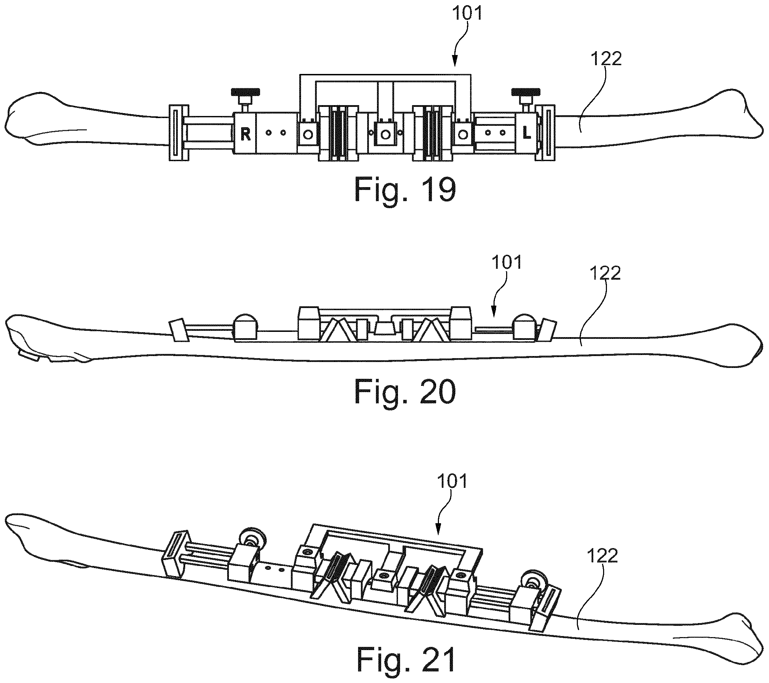

Der Vorgang des Aufsetzens, Schneidens und Entnehmens sowie nachfolgenden Zusammensetzens ist den Fign. 19 bis 38 gut zu entnehmen.

### Bezugszeichenliste

- 1: Unterkieferresektionsschablone
- 2: Zentralteil / Zentralbauteil
- 3: Trennwerkzeugführabschnitt / Sägeblattführabschnitt
- 4: Positionierhilfe / Positionierflosse
- 5: Horizontalsteg
- 6: oberer Horizontalsteg
- 7: unterer Horizontalsteg
- 8: distales Ende des Horizontalsteges
- 9: Block
- 10: Sägeschlitz
- 11: Knochentrennwerkzeuganlagefläche
- 12: Markierung
- 13: Oberfläche der Positionierflosse
- 14: Erweiterungsbauteil
- 15: Grundkörper
- 16: Zusatzsägeblattführabschnitt
- 17: Verstellmechanismus
- 18: Durchgangsschlitz
- 19: Unterkieferauflagebock
- 20: Schlitzverlängerung
- 21: Stab
- 22: Frontoberfläche
- 23: Riffelung
- 24: Kanal
- 25: Arretiermittel
- 26: Durchgangsloch
- 27: Unterkiefer
- 101: Fibula-Knochenmaterialentnahme- und -verbringschablone
- 102: Mittelteil
- 103: Ende
- 104: Knochentrennwerkzeugführabschnitt
- 105: Vertikalfläche
- 106: Führungsschlitz
- 107: Balken
- 108: Bügelaufnahmevorrichtung
- 109: Klammer
- 110: Durchgangsloch
- 111: Federabschnitt
- 112: Entnahmehilfsbügel
- 113: Aufnahmeloch
- 114: Ergänzungsbauteil
- 115: Zentralkörper
- 116: Verschiebemechanismus
- 117: Führungssteg
- 118: Frontseite
- 119: Rasterung
- 120: Verstellschraube
- 121: Knochenschraube
- 122: Fibula-Knochen
- 123: Implantierhilfsbügel

## Patentansprüche

1. Unterkieferresektionsschablone (1) mit einem Zentralbauteil (2), das zum Anbringen an einem Segment, etwa einem Symphysensegment eines Kieferknochens, wie eines Unterkiefers oder eines Oberkiefers, vorbereitet ist, wobei an dem Zentralbauteil (2) mindestens zwei Trennwerkzeugführabschnitte (3) vorhanden sind, und wobei zwischen den beiden oder mehreren Trennwerkzeugführabschnitten (3) eine Positionierhilfe (4) vorhanden ist, um eine raumkorrekte Ausrichtung der Unterkieferresektionsschablone (1) zum Kieferknochen zu bewerkstelligen, wobei das Zentralbauteil (2) als Rahmen mit zwei Horizontalstegen (5, 6, 7) ausgebildet ist, **dadurch gekennzeichnet, dass** und an einer Vorderseite und/oder einer Oberseite zumindest eines Horizontalsteges (5, 6, 7) eine Mittellinienmarkierung (12) vorhanden ist.

2. Unterkieferresektionsschablone (1) nach Anspruch 1, **dadurch gekennzeichnet, dass** die Positionierhilfe (4) als eine Kimme-Korn-Kombination, eine Kerbe, eine Bohrung, ein Prisma, eine Farbmarkierung, ein Stift, eine Schraube oder eine Navigationspositionierung ausgebildet ist und/oder als eine Positionierflosse zum in Kontakt gelangen mit einem Abschnitt des Unterkieferknochens (27) vorbereitete Positionierflosse (4) ausgebildet ist.

3. Unterkieferresektionsschablone (1) nach Anspruch 1 oder 2, **dadurch gekennzeichnet, dass** die Positionierhilfe (4) vom Zentralbauteil (2) quer absteht.

4. Unterkieferresektionsschablone (1) nach einem der Ansprüche 1 bis 3, **dadurch gekennzeichnet, dass** die zwei Horizontalstege (5, 6, 7) an ihren distalen Enden (8) über die beiden als Sägeblattführabschnitte ausgebildeten Trennwerkzeugführabschnitte (3) miteinander verbunden sind.

5. Unterkieferresektionsschablone (1) nach Anspruch 4, **dadurch gekennzeichnet, dass** die beiden Trennwerkzeugführabschnitte (3) und die beiden Horizontalstege (5, 6, 7) zumindest in einer Frontalprojektion eine rechteckige Form besitzen.

6. Unterkieferresektionsschablone (1) nach einem der Ansprüche 4 oder 5, **dadurch gekennzeichnet, dass** die beiden Horizontalstege (5, 6, 7) und die beiden Trennwerkzeugführabschnitte (3) als ein einheitliches und/oder einstückiges / integrales und/oder einmaterialiges Bauteil ausgeformt sind.

7. Unterkieferresektionsschablone (1) nach einem der Ansprüche 1 bis 6, **dadurch gekennzeichnet, dass** zumindest einer der Trennwerkzeugführabschnitte (3) nach Art eines Sägeblattführabschnitts einen Sägeschlitz (10) besitzt, der zum Führen eines Knochentrennwerkzeuges dimensioniert ist.

8. Unterkieferresektionsschablone (1) nach Anspruch 7, **dadurch gekennzeichnet, dass** beidseitig des Sägeschlitzes (10) eine zu diesen parallele Knochentrennwerkzeuganlagefläche (11) ausgebildet ist.

9. Unterkieferresektionsschablone (1) nach Anspruch 7 oder 8, **dadurch gekennzeichnet, dass** der Sägeschlitz (10) vollständig durch das ihn ausbildende Material hindurch ragt und zusätzlich in Verlängerung seiner Längserstreckung einseitig oder zweiseitig offen ausgestaltet ist.

10. Unterkieferresektionsschablone (1) nach Anspruch 9, **dadurch gekennzeichnet, dass** das den Sägeschlitz (10) beherbergende Material blockartig ausgestaltet ist und/oder einer der Horizontalstege (5, 6, 7) rechteckbalkenartig ausgebildet ist.

## Claims

1. A mandibular resection template (1) comprising a central component (2) which is prepared for attachment to a segment, for example a symphysis segment of a jawbone, such as a mandible or a maxilla, wherein at least two separating tool guide portions (3) are present at the central component (2), and wherein a positioning aid (4) is provided between the two or more separating tool guide portions (3) in order to obtain a spatially correct orientation of the mandibular resection template (1) with respect to the jawbone, wherein the central component (2) is in the form of a frame having two horizontal webs (5, 6, 7), **characterized in that** on a front side and/or an upper side of at least one of the horizontal webs (5, 6, 7) a center line marker (12) is present.

2. The mandibular resection template (1) according to claim 1, **characterized in that** the positioning aid (4) is configured as notch and bead combination, notch, bore, prism, color marker, pin, screw or navigation positioning and/or as a positioning fin (4) prepared for entering into contact with a portion of the mandibular bone (27).

3. The mandibular resection template (1) according to claim 1 or 2, **characterized in that** the positioning aid (4) projects transversely from the central component (2).

4. The mandibular resection template (1) according to one of the claims 1 to 3, **characterized in that** the two horizontal webs (5, 6, 7) are connected to each other at their distal ends (8) via the two separating tool guide portions (3) configured as saw blade guide portions.

5. The mandibular resection template (1) according to claim 4, **characterized in that** the two separating tool guide portions (3) and the two horizontal webs (5, 6, 7) have a rectangular shape at least in a frontal projection.

6. The mandibular resection template (1) according one of the claims 4 to 5, **characterized in that** the two horizontal webs (5, 6, 7) and the two separating tool guide portions (3) are configured as a uniform and/or one-piece/integral and/or single-material component.

7. The mandibular resection template (1) according to one of the claims 1 to 6, **characterized in that** at least either of the separating tool guide portions (3) includes a saw slit (10) in the form of a saw blade guide portion which is dimensioned to guide a bone separating tool.

8. The mandibular resection template (1) according to claim 7, **characterized in that** on both sides of the saw slit (10), a bone separating tool contact face (11) parallel thereto is formed.

9. The mandibular resection template (1) according to claim 7 or 8, **characterized in that** the saw slit (10) protrudes completely through the material forming it and, in addition, is configured in extension of its longitudinal extension to be open on one side or on two sides.

10. The mandibular resection template (1) according to claim 9, **characterized in that** the material accommodating the saw slit (10) is block-shaped and/or one of the horizontal webs (5, 6, 7) is in the form of a rectangular beam.

## Revendications

1. Gabarit de résection de mâchoire inférieure (1) avec un composant central (2) qui est préparé pour être appliqué à un segment, tel qu'un segment de symphyse d'un os de la mâchoire, comme une mâchoire inférieure ou une mâchoire supérieure,
dans lequel au moins deux sections de guidage d'outil de séparation (3) sont prévues sur le composant central (2), et dans lequel une aide de positionnement (4) est prévue entre les deux ou plusieurs sections de guidage d'outil de séparation (3) pour obtenir un alignement spatialement correct du gabarit de résection de mâchoire inférieure (1) avec l'os de la mâchoire, dans lequel le composant central (2) est conçu comme un cadre avec deux traverses horizontales (5, 6, 7), **caractérisé en ce qu'**un marquage de ligne centrale (12) est présent sur un côté avant et/ou un côté supérieur d'au moins une traverse horizontale (5, 6, 7).

2. Gabarit de résection de mâchoire inférieure (1) selon la revendication 1, **caractérisé en ce que** l'aide de positionnement (4) est formée comme une combinaison mire/visée, une encoche, un alésage, un prisme, un marquage de couleur, un goujon, une vis ou un positionnement de navigation et/ou est formée comme une ailette de positionnement pour entrer en contact avec une section de l'ailette de positionnement (4) préparée de la mâchoire inférieure (27).

3. Gabarit de résection de la mâchoire inférieure (1) selon la revendication 1 ou 2, **caractérisé en ce que** l'aide de positionnement (4) fait saillie transversalement à partir du composant central (2).

4. Gabarit de résection de mâchoire inférieure (1) selon l'une quelconque des revendications 1 à 3, **caractérisé en ce que** les deux traverses horizontales (5, 6, 7) sont reliées entre elles à leurs extrémités distales (8) par l'intermédiaire des deux sections de guidage d'outil de séparation (3) conçues comme des sections de guidage de lame de scie.

5. Gabarit de résection de mâchoire inférieure (1) selon la revendication 4, **caractérisé en ce que** les deux sections de guidage d'outil de séparation (3) et les deux traverses horizontales (5, 6, 7) ont une forme rectangulaire au moins en projection frontale.

6. Gabarit de résection de mâchoire inférieure (1) selon l'une quelconque des revendications 4 ou 5, **caractérisé en ce que** les deux traverses horizontales (5, 6, 7) et les deux sections de guidage d'outil de séparation (3) sont formées comme un composant unitaire et/ou d'une seule pièce/d'un seul tenant et/ou d'un seul matériau.

7. Gabarit de résection de la mâchoire inférieure (1) selon l'une quelconque des revendications 1 à 6, **caractérisé en ce qu'**au moins une des sections de guidage d'outil de séparation (3) présente une fente de sciage (10) à la manière d'une section de guidage de lame de scie, qui est dimensionnée pour le guidage d'un outil de séparation d'os.

8. Gabarit de résection de mâchoire inférieure (1) selon la revendication 7, **caractérisé en ce qu'**une surface d'appui d'outil de séparation d'os (11), parallèle à la fente de sciage (10), est formée des deux côtés de celle-ci.

9. Gabarit de résection de mâchoire inférieure (1) selon la revendication 7 ou 8, **caractérisé en ce que** la fente de sciage (10) fait entièrement saillie à travers le matériau qui la forme et est en outre ouverte sur un ou deux côtés dans le prolongement de son extension longitudinale.

10. Gabarit de résection de mâchoire inférieure (1) selon la revendication 9, **caractérisé en ce que** le matériau recevant la fente de sciage (10) est conçu en forme de bloc et/ou l'une des traverses horizontales (5, 6, 7) est conçue en forme de poutre rectangulaire.
